# EUROPÄISCHE PATENTSCHRIFT

(11) **EP 1 141 155 B1**
(45) Veröffentlichungstag und Bekanntmachung des Hinweises auf die Patenterteilung: **23.04.2014**
(21) Anmeldenummer: 99963523.8
(22) Anmeldetag: 14.12.1999
(51) Int. Cl.: C09J 4/00, C08F 222/32, C08K 5/378, C09J 5/02

(54) **AKTIVATOR FÜR CYANACRYLAT-KLEBSTOFFE**
ACTIVATOR FOR CYANACRYLATE ADHESIVES
ACTIVATEUR DE COLLES AU CYANACRYLATE

(30) Priorität: 23.12.1998 DE 19859638
(43) Veröffentlichungstag der Anmeldung: 10.10.2001
(73) Patentinhaber: Henkel AG & Co. KGaA, 40589 Düsseldorf (DE)
(72) Erfinder: MISIAK, Hanns, Roland, D-33332 Gütersloh (DE); SCHEFFLER, Ingolf, D-41470 Neuss (DE)
(86) Internationale Anmeldenummer: PCT/EP1999/009896
(87) Internationale Veröffentlichungsnummer: WO 2000/039229

(56) Entgegenhaltungen:
- DD-A- 278 033
- DE-A- 1 719 161
- DE-A- 2 261 261

## Beschreibung

Die Erfindung betrifft Aktivatoren zur beschleunigten Aushärtung von Cyanacrylatklebstoffen sowie ein Verfahren zur beschleunigten Verklebung von Substraten mit Hilfe von Cyanacrylat-Klebstoffen.

Klebstoffe, die einen oder mehrere Ester der 2-Cyanoacrylsäure enthalten, zeichnen sich bekanntermaßen dadurch aus, daß sie sich zum Verkleben zweier gegenüberliegender Flächen oder Träger eignen, insbesondere wenn die Verklebung rasch aushärten soll. Die Ester der 2-Cyanoacrylsäure, auch kurz als Cyanacrylat-Klebstoffe bekannt, werden im allgemeinen Sprachgebrauch wegen ihres normalerweise sehr schnellen Abbindeverhaltens auch "Sekundenkleber" genannt, da sie in der Regel bei der Anwendung nach wenigen Sekunden aushärten oder die gefügten Teile zumindest eine gewisse Anfangsfestigkeit zeigen. Bekanntermaßen wird das Abbinden von Cyanacrylat-Klebstoffen durch eine anionische Polymerisationsreaktion ausgelöst. Bei einigen Substraten, insbesondere bei sauren Substraten wie z. B. Holz oder Papier kann diese Polymerisationsreaktion jedoch sehr stark verzögert werden. Darüber hinaus zeigen die beiden genannten Materialien eine ausgeprägte Tendenz, den oft sehr dünnflüssigen Klebstoff durch Kapillarwirkung aus dem Fügespalt zu saugen, bevor in diesem die Härtung erfolgt ist. Auch in Fällen, in denen, geometriebedingt, der Klebstoff in der Klebefuge in dickerer Schicht appliziert werden muß oder in Fällen, wo absichtlich oder unabsichtlich relativ große Mengen Klebstoff derart appliziert werden, daß z. B. größere Tropfen des Klebstoffes zwischen den zu fügenden Teilen hervorquellen, ist eine schnelle durchgehende Härtung kaum zu erreichen.

Es hat bereits zahlreiche Versuche gegeben, für derartige Anwendungsfälle die Beschleunigung der Polymerisation durch spezielle Zusätze zu bewirken. Diese Vorgehensweisen lassen sich grob in drei Kategorien einteilen:
- Zusatz von Beschleunigern direkt zur Klebstoff-Formulierung. Dies ist jedoch nur in einem sehr begrenzten Umfang möglich, da basisch oder nukleophil wirkende Substanzen, die normalerweise eine starke Beschleunigung der Polymerisation des Cyanacrylat-Klebstoffes bewirken würden, in aller Regel zu Lasten der Lagerstabilität derartiger Zusammensetzungen gehen.
- Der zweite Weg, der allgemein beschritten wird, ist der Zusatz der obengenannten Beschleuniger kurz vor der Applikation des Klebstoffes in einem quasi Zweikomponentensystem. Diese Vorgehensweise hat jedoch den Nachteil der begrenzten Topfzeit nach der Einmischung des Aktivators. Außerdem ist bei den geringen notwendigen Aktivatormengen die notwendige Dosiergenauigkeit und Mischhomogenität schwer zu erzielen.
- Ein drittes allgemein angewandtes Verfahren ist die Verwendung von Aktivatoren in Form einer verdünnten Lösung. Dabei wird diese entweder vorher auf die zu verklebenden Teile aufgesprüht oder nach der Fügung der Substrate auf die Stellen mit noch flüssigem Klebstoff aufgesprüht. Als Lösungsmittel für derartige verdünnte Lösungen von Aktivatoren werden in der Regel niedrigsiedende organische Lösungsmittel verwendet.

Für den ersten Lösungsweg wurden beispielsweise in der JP-A-10140091 die Verwendung von Kronenethern oder Polyalkylenoxiden als Härtungsbeschleunigungszusatz in Cyanacrylatklebstoff-Mischungen vorgeschlagen. Die DE-A-40 09 621 schlägt die Verwendung von speziellen, teilweise in Cyanacrylaten löslichen Cyclodextrinderivaten als Zusatz vor. Die US-A-4 718 966 schlägt den Zusatz von Calixaren-Verbindungen als additiven Beschleuniger in Cyanacrylat-Klebstoffzusammensetzungen vor, die GB-A-2 200 124 schlägt den Einsatz von acyclischen Phenol-Formaldehyd-Oligomeren als Beschleunigungszusatz zu Cyanacrylat-Klebstofformulierungen vor.

In der US-A-4 460 759 werden zweikomponentige Klebstoffzusammensetzungen vorgeschlagen, in der die eine Komponente das Cyanacrylat-Monomer enthält und die zweite Komponente einen schwach sauren oder schwach basischen ionischen Beschleuniger, bestehend aus einem Kation mit einem pKa von mindestens 10 und einem nukleophilen Anion.

Für den dritten Weg, die Verwendung von Beschleunigerlösungen, schlägt die JP-A-62 022 877 die Verwendung von Lösungen von niedrigen Fettaminen, aromatischen Aminen, Dimethylamin und dergleichen vor. Die JP-A-03 207 778 schlägt die Verwendung von Lösungen aliphatischer, alicylklischer und insbesondere tertiärer aromatischer Amine vor, konkret genannt werden N, N-Dimethylbenzylamin, N-Methylmorpholin und N,N-Diethyltoluidin.

Die letztgenannte Verbindung, das N-N-Dimethyl-p-toluidin ist praktisch die einzige seit langem weltweit fast ausschließlich eingesetzte Verbindung als Beschleunigersubstanz bei beschleunigter Aushärtung von Cyanacrylatklebstoffen. Bei nachträglicher Applikation auf die gefügten Teile bewirkt die Substanz eine innerhalb von Sekunden ablaufende Härtung auch größerer Mengen an Klebstoff. Das so entstandene Polycyanacrylat ist völlig frei von Trübungen. Entscheidende Nachteile bei der Verwendung dieser Substanz liegen in der recht hohen Flüchtigkeit der Substanz, die keine langen Wartezeiten zwischen dem vorherigen Aufbringen der Beschleunigerlösung auf die zu verklebenden Substrate und dem anschließenden Verklebungsvorgang erlauben. Ein weiterer entscheidender Nachteil ist die ausgesprochene Toxizität dieser Verbindung, die nach dem Chemikaliengesetz mit "T" gekennzeichnet werden muß.

Die Erfahrung hat gezeigt, daß die Basizität oder Nukleophilie der Beschleunigersubstanzen als alleiniges Selektionskriterium nicht ausreicht, um in der Praxis anwendungstechnisch akzeptable Problemlösungen zu erzielen. Viele Substanzen, wie z. B. Alkylamine, Triphenylphosphan, 1,2-Di-(4-pyridyl-ethan), 4,4'-Dipyridyl-disulfid, 3-(3-Hydroxypropyl)pyridin, 1,2-Bis(diphenylphosphino)-ethan, Pyridazin, Methylpyridazin oder 4,4'-Dipyridyl sind derart basisch bzw. nukleophil, daß eine spontane oberflächliche Härtung an der Klebstoffgrenzfläche eintritt (Schockhärtung), bevor der Aktivator durch Konvektion und Diffusion eine durchgehende Polymerisation der flüssigen Klebstoffschicht auslösen kann. Bei anderen Verbindungen wie z. B. Oxazolen ist die Basizität offensichtlich zu gering. Das Ergebnis ist im Fall der Schockhärtung eine oft trübe, nur auf der Oberfäche ablaufende Härtung, im Falle der letztgenannten Verbindung ist die Aushärtung für die Praxis viel zu langsam.

Die DE-A-22 61 261 schlägt Beschleunigersubstanzen vor, die das Strukturelement -N=C-S- enthalten. Testreihen mit Verbindungen, die diese Atomgruppierung aufweisen, zeigen tatsächlich, daß eine relativ schnelle und sichere Aushärtung auch größerer Klebstoffmengen erreicht wird. Im Vergleich mit dem oben erwähnten "Klassiker", N,N-Dimethyl-p-toluidin ist der aktivierende Effekt bei fast allen leicht zugänglichen Substanzen gemäß DE-A-22 61 261 jedoch deutlich weniger ausgeprägt, so daß signifikant längere Wartezeiten für die Abbindung in Kauf genommen werden müssen. Einzig das 2,4-Dimethylthiazol zeigt eine sehr gute beschleunigende Wirkung. Diese Verbindung weist jedoch eine sehr hohe Flüchtigkeit auf, so daß Aktivatorlösungen auf Basis dieser Verbindung für eine vorherige Applikation ungeeignet sind, da mit dem Lösungsmittel auch der Wirkstoff abdampft.

Es bestand also die Aufgabe, neue Aktivatorsubstanzen für die Verwendung bei Verklebungen von Cyanacrylat-Klebstoffen zu finden, die eine stark beschleunigende Wirkung haben und eine geringe Flüchtigkeit, so daß auch eine vorherige Applikation möglich ist. Außerdem sollte diese Verbindung arbeitshygienisch unbedenklicher sein als das z. Zt. noch in großem Umfange eingesetzte N,N-Dimethyl-p-tofuidin.

Die erfindungsgemäße Lösung der Aufgabe ist den Patentansprüchen zu entnehmen. Sie besteht im wesentlichen darin, daß Aktivatoren zur Zubereitung von Aktivatorlösungen eingesetzt werden, die das Strukturelement - N=C-S-S- oder das Strukturlement -N=C-S-S-C=N- enthalten, wobei die N=C-Doppelbindung nicht notwendigerweise eine typische Doppelbindung sein muß, sondern auch Teil eines aromatischen π-Systems sein kann. Konkrete Vertreter für derartige Verbindungen sind das Dibenzodiazyldisulfid, 6,6'-Dithiodinicotinsäure, das 2,2'-Dipyridyldisulfid oder das Bis-(4-t-butyl-1-isopropyl-2-imidazolyl)-disulfid. Erfindungsgemäß werden die Aktivatoren in leicht flüchtigen Lösungsmitteln wie z. B. Kohlenwasserstoffen, Carbonsäureestern, Ketonen, Ethern oder auch Halogenkohlenwasserstoffen gelöst. Diese Lösungen der Aktivatoren enthalten die Aktivator-Verbindung in Konzentrationen zwischen 0,01 g bis 10 g pro 100 ml Lösungsmittel, vorzugsweise sind 0,1 bis 5 g Aktivatorsubstanz pro 100 ml Lösungsmittel gelöst.

Als Lösungsmittel für die erfindungsgemäßen Aktivatoren eignen sich eine Vielzahl gebräuchlicher organischer Lösungsmittel, solange sie eine ausreichend hohe Flüchtigkeit haben. Dies bedeutet, daß der Siedepunkt des Lösungsmittels unter 120°C, vorzugsweise unter 100°C bei Normaldruck sein sollte. Konkrete Beispiele für geeignete Lösungsmittel sind Siedegrenzenbenzine, insbesondere jedoch das n-Heptan, Alkylester von niederen Carbonsäuren beispielsweise Ethylacetat, lsopropylacetat, Butylacetat, Ketone wie beispielsweise Aceton, Methylisobutylketon, Methylethylketon. Weiterhin sind geeignet Etherlösungsmittel, Etherester oder cyclische Ether, wie insbesondere das Tetrahydrofuran. Bei andererseits schwer löslichen Aktivatoren können jedoch auch chlorierte Kohlenwasserstoffe wie Dichlormethan oder Trichlormethan (Chloroform) eingesetzt werden.

Die erfindungsgemäßen Aktivatorlösungen eignen sich zum beschleunigten Aushärten für alle herkömmlichen Cyanacrylat-Klebstoffe, die als wesentlichen Bestandteil einen oder mehrere Cyanacrylsäureester, Inhibitoren gegen radikalische Polymerisation, Inhibitoren gegen anionische Polymerisation und gegebenenfalls übliche in derartigen Klebstoffsystemen eingesetzten Hilfsstoffe enthalten.

Die in den Klebstoffen eingesetzten Cyanacrylsäureester sind in der Hauptsache ein oder mehrere Ester der 2-Cyanacrylsäure. Diese Ester haben die allgemeine Formel:

H₂C = C(CN)-CO-O-R.

In ihr ist R eine Alkyl-, Alkenyl-, Cycloalkyl-, Aryl-, Alkoxyalkyl-, Aralkyloder Haloalkylgruppe, insbesondere eine Methyl-, Ethyl-, n-Propyl-, isoPropyl-, n-Butyl-, iso-Butyl-, Pentyl-, Hexyl-, Allyl-, Methallyl-, Crotyl-, Propargyl-, Cyclohexyl-, Benzyl-, Phenyl-, Cresyl-, 2-Chlorethyl-, 3-Chlorpropyl-, 2-Chlorbutyl-, Trifluorethyl-, 2-Methoxyethyl-, 3-Methoxybutylund 2-Ethoxyethylgruppe. Die vorgenannten Cyanoacrylate sind dem Klebstoff-Fachmann bekannt, vgl. Ullmann's Encyclopaedia of Industrial Chemistry, Band. A1, S. 240, Verlag Chemie Weinheim (1985) sowie US-PS 3 254 111 und US-PS 3 654 340. Bevorzugte Monomere sind die Allyl-, Methoxyethyl-, Ethoxyethyl-, Methyl-, Ethyl-, Propyl-, Isopropyl- oder ButylEster der 2-Cyanoacrylsäure. Die Monocyanoacrylsäureester stellen den größten Gewichtsanteil der polymerisierbaren Monomeren im Klebstoff dar.

Die genannten Cyanacrylsäureester sind in den Klebstoffen in Mengen von 99,99 bis 90 Gew.-% enthalten. Bevorzugt sind Cyanacrylsäureester, deren Alkoholrest sich von Alkoholen mit 1 bis 10 C-Atomen ableitet, die auch cyclisch, verzweigt oder perfluoriert sein können.

Die erfindungsgemäßen Cyanacrylklebstoffe können weiterhin einen Inhibitor für die radikalische Polymerisation enthalten. Solche Inhibitoren sind zum Beispiel Hydrochinon, p-Methoxyphenol, aber auch sterisch gehinderte Phenole, Phenothiazin und dergleichen.

Als weitere Hilfsstoffe können die erfindungsgemäßen Cyanacrylatklebstoffe auch Verdickungsmittel enthalten. Dies ist insbesondere dann gewünscht, wenn poröse Materialien verklebt werden sollen, die ansonsten den niedrig viskosen Klebstoff leicht aufsaugen. Als Verdicker können viele Arten von Polymeren verwendet werden, so zum Beispiel Polymethylmethacrylat, andere Methacrylatcopolymere, Acrylkautschuk, Cellulosederivate, Polyvinylacetat oder Polyalphacyanacrylat. Eine übliche Menge an Verdicker ist im allgemeinen ungefähr 10 Gew.-% oder weniger, bezogen auf den Gesamtklebstoff. Neben oder anstelle der Verdicker können die erfindungsgemäßen Cyanacrylatklebstoffe auch noch Verstärker enthalten. Beispiele für solche Verstärker sind Acrylelastomere, Acrylnitrilcopolymer, Elastomere oder Fluorelastomere. Darüber hinaus können auch anorganische Zusätze zum Beispiel Silikate, Thixotropiermittel mit hoher Oberfläche, die vorzugsweise mit Polydialkylsiloxanen beschichtet sind, eingesetzt werden.

Die erfindungsgemäßen Cyanacrylatklebstoffe können weiterhin Stoffe zur Steigerung der thermischen Stabilität enthalten. Hierzu können beispielsweise die in EP 579 476 genannten Schwefelverbindungen eingesetzt werden.

Neben oder anstelle der genannten Additive können die erfindungsgemäßen Cyanacrylatklebstoffe auch noch Weichmacher enthalten. Diese dienen dazu, die erhaltene Klebeverbindung gegen Brüchigkeit zu schützen. Solche Weichmacher sind hier beispielsweise C₁-bis C₁₀-Alkylester zweibasischer Säuren, insbesondere der Sebacinsäure, Phthalsäure oder der Malonsäure. Andere Weichmacher sind Diarylether und Polyurethane und dergleichen. Schließlich können die erfindungsgemäßen Klebstoffzubereitungen auch noch Farbstoffe, Pigmente, Geruchsstoffe, Extender und dergleichen enthalten.

Die Erfindung soll nun anhand von einigen Beispielen näher erläutert werden.

### Beispiele

Zur Überprüfung der Wirksamkeit der Aktivatorlösungen und anderer basischer, aktivierender Verbindungen wurde die Aushärtungsgeschwindigkeit an einem handelsüblichen Cyanacrylatklebstoff auf Aluminiumblechen getestet (hochviskoses Sicomet 63 der Firma Sichel-Werke, Hannover). Aufgetragen wurde jeweils eine Raupe mit Volumen 0.25 ml der Ausmasse 9 cm x 0,8 cm. Ergebnisse siehe Tabelle 1.
Applikation des Aktivators: 1 Sekunde aus Sprayflasche; 35 cm Sprühabstand.

**Tabelle 1; Aushärtungszeiten von Cyanacrylatklebstoff nach Anwendung verschiedener die Polymerisation auslösender Verbindungen:**

| **Beispiel** | **Aktivator** | **Lösung** | **Härtungsge-schwindigkeit/ Bemerkungen** |
|---|---|---|---|
| **1** | 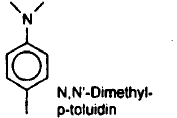 | 0.4 g / 100 ml n-Heptan | 15s |
| **2** | 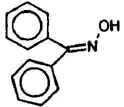 | 0.4 g / 100 ml Ethylacetat | 60 s |
| | | | (Spontane oberflächliche Aushärtung; völlige Durchhärtung aber erst nach ca. 60 s) |
| **3** | 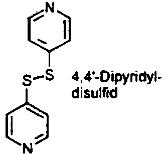 | 0.4 g / 100 ml Ethylacetat | (Spontane, trübe obertlächliche Aushärtung; völlige Durchhärtung aber erst nach etlichen Minuten) |
| **4** | 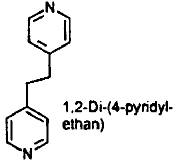 | 0.4 g / 100 ml Ethylacetat | (Spontane, trübe oberflächlich Aushärtung; völlige Durchhärtung aber erst nach etlichen Minuten) |
| **5** | 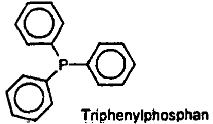 | 0.4 g / 100 ml Ethylacetat | (Spontane, trübe oberflächliche Aushärtung; völlige Durchhärtung aber erst nach etlichen Minuten) |
| **6** | 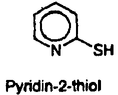 | 0.4 g / 100 ml Ethylacetat | ca. 180 s |
| **7** | 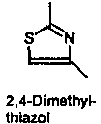 | 0.4 g / 100 ml Ethylacetat | 5 s |
| **8** | 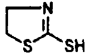 | 0.4 g / 100 ml Ethylacetat | 90 s |
| **9** | 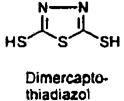 | 0.4 g / 100 ml Ethylacetat | ca. 300 s |
| **10** | 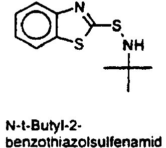 | 0.4 g / 100 ml n-Heptan | 45 s |
| **11** | | 0.4 g / 100 ml Ethylacetat | 50 s |
| **12** | 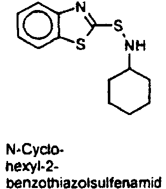 | 0.4 g / 100 ml n-Heptan | 50 s |
| **13** | | 0.4 g / 100 ml Ethylacetat | 50 s |
| **14** | 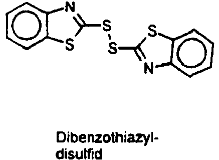 | 0,4 g / 100 ml Trichlormethan | 15s |
| **15** | | 0,4 g / 100 ml Dichlormethan | 15s |
| **16** | 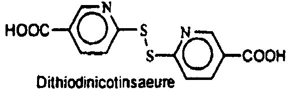 | 0,4 g /100 ml Tetrahydrofuran | 30 s |
| **17** | 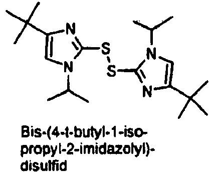 | 0,4 g / 100 ml Dichlormethan | 15s |
| **18** | 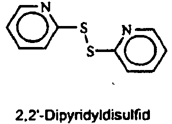 | 0.4 g / 100 ml n-Heptan | 10s |
| **19** | | 0.4 g / 100 ml Ethylacetat | 15s |

**Tabelle 2: Abbindegeschwindigkeit an Aluminiumstreifen (entfettet; 10 x 2 cm) nach entsprechender Wartezeit (nach Abdunsten des Lösungsmittels) (verwendet wurde der viskose CA-Klebstoff Sicomet 63):**

| **Verwendete Lösung (siehe Tabelle 1)** | **Abbindezeit nach 5 Minuten Wartezeit** | **Abbindezeit nach 10 Minuten Wartezeit** |
|---|---|---|
| **1** | **>60 s** | **>60s** |
| **7** | **>60 s** | **>60s** |
| **10** | **1 s** | **2 s** |
| **12** | **1 s** | **1 s** |
| **14** | **1 s** | **2 s** |
| **16** | **10 s** | **12 s** |
| **17** | **1 s** | **1 s** |
| **18** | **3 s** | **3 s** |
| **Ohne Vorbehandiung** | **> 60 s** | **> 60 s** |

Die Beispiele 1 bis 13 sind nicht erfindungsgemäße Vergleichsbeispiele. Dabei ist das Beispiel 1 der gängige weit verbreitete Aktivator gemäß Stand der Technik. Die Beispiele 2 bis 5 zeigen, daß eine Vielzahl von aktivierend wirkenden Substanzen für die Praxis ungeeignet sind, da sie zwar zu einer spontanen, oberflächlichen Aushärtung führen, die völlige Durchhärtung aber sehr lange Zeit in Anspruch nimmt. Die Aktivatoren gemäß den Beispielen 6 bis 13 entsprechen der Lehre der DE-A-22 61 261. Aus der Tabelle 1 wird deutlich, daß die Aktivatoren der Beispiele 6 und 8 bis 13 eine nur leichte beschleunigende Wirkung aufweisen; der Aktivator des Beispiels 7 weist zwar eine sehr hohe Abbindegeschwindigkeit auf, wie aus der Tabelle 2 ersichtlich, erlaubt dieser Aktivator jedoch keine verlängerte Wartezeit zwischen Auftrag des Aktivators (nach Abdunsten des Lösungsmittels) und Klebstoffauftrag und Fügen der Klebeverbindung.

Die erfindungsgemäßen Aktivatoren gemäß Beispielen 14 bis 19 weisen sowohl eine starke beschleunigende Wirkung auf (dokumentiert in sehr hoher Härtungsgeschwindigkeit, siehe Tabelle 1), gleichzeitig erlauben sie eine lange Wartezeit zwischen Auftrag des Aktivators und Klebstoffauftrag (siehe Tabelle 2).

Im Gegensatz zu den Aktivatoren des Standes der Technik, die entweder eine geringe beschleunigende Wirkung haben oder eine ausreichend beschleunigende Wirkung haben jedoch eine zu hohe Flüchtigkeit aufweisen, zeigen die erfindungsgemäß beschriebenen Substanzen diese Nachteile nicht, denn sie zeigen folgendes Eigenschaftsprofil:
- Aushärtung erfolgt in einigen Sekunden. Sie ist in der Regel schneller als in den die Atomgruppierung -N=C-S- enthaltenden Verbindungen (siehe DE-A- 22 61 261). In diesem Zusammenhang ist ein Vergleich der aus den Beispielen 6, Pyridin-2-thiol und 18/19, beide aus Tabelle 1, interessant: Das 2,2'-Dipyridyldisulfid mit dem Strukturelement -N=C-SS-C=N- ist optimal aktivierend und ist chemisch dem Pyridin-2-thiol, mit -N=C-S-Gruppierung, sehr ähnlich, welches aber deutlich weniger aktivierend ist (2,2'-Dipyridyldisulfid entspricht dem durch milde Oxidation von Pyridin-2-thiol leicht zugänglichen Reaktionsprodukt).
- Die Aushärtung ist nicht zu schnell; d. h. spröde Schockhärtung wird vermieden und somit:
- Polymerisiertes Cyanacrylat ist klar, transparent, nicht trübe.
- Die Verbindungen in Form ihrer Lösungen können auch vor dem Verkleben der Fügeteile aufgetragen werden. Auch nach mehreren Minuten Wartezeit sind die Materialoberflächen noch aktiviert.

## Patentansprüche

1. Aktivatorlösung zum beschleunigten Aushärten von CyanacrylatklebStoffen, **dadurch gekennzeichnet, daß** der Aktivator in einem Lösungsmittel mit einem Siedepunkt unter 120°C gelöst ist und der Aktivator eine organische Verbindung ist, die das Strukturelement -N=C-S-S- enthält.

2. Aktivatorlösung nach Anspruch 1, **dadurch gekennzeichnet, daß** die organische Verbindung das Strukturelement -N=C-S-S-C=N- enthält.

3. Aktivatorlösung nach Anspruch 2, **dadurch gekennzeichnet, daß** der Aktivator ausgewählt wird aus Dibenzothiazyldisulfid, 6,6'-Dithiodinicotinsäure, 2,2'-Dipyridyldisulfid, Bis-(4-t-butyl-1-isopropyl-2-imidazolyl)-disulfid.

4. Aktivatorlösung nach mindestens einem der vorhergehenden Ansprüche, **dadurch gekennzeichnet, daß** als Lösungsmittel flüchtige Kohlenwasserstoffe, Carbonsäureester, Ketone, Ether oder Halogenkohlenwasserstoffe verwendet werden.

5. Aktivatorlösung nach mindestens einem der vorhergehenden Ansprüche, **gekennzeichnet durch** eine Aktivatorkonzentration von 0,01 g bis 10 g, vorzugsweise 0,1 g bis 5 g Aktivatorsubstanz pro 100 ml Lösungsmittel.

6. Verfahren zum Verkleben von Substraten, **gekennzeichnet durch** die folgenden wesentlichen Verfahrensschritte
a) Sprühen der Aktivatorlösung nach Anspruch 1 bis 5 auf mindestens eine Kiebefläche der zu verbindenden Substrate bzw. Fügeteile
b) Ablüften des Lösungsmittels, gegebenenfalls unter Erwärmen oder mit Hilfe eines Gebläses
c) Aufbringen des Cyanacrylat-Klebstoffes auf mindestens ein Substratteil
d) Fügen der zu verbindenden Substratteile gegebenenfalls unter
kurzfristigem mechanischem Fixieren
e) Gegebenenfalls nachträgliches Aufsprühen der Aktivatorlösung, nach Anspruch 1 bis 5 auf überstehenden oder exzessiv verwendeten oder aus dem Fügespalt austretenden Klebstoff.

7. Verfahren nach Anspruch 6, jedoch ohne Schritt b).

8. Verfahren nach Ansprüche 6 oder 7, mit dem Unterschied, daß in Schritt a) die Aktivatorslösung zuvor mit Hilfe eines Pinsels oder Rührstabes oder Spatel auf die Fügeteile aufgetragen wird.

9. Verfahren nach Anspruch 8, mit dem Unterschied, daß in Schritt a) die zu verklebende Fügeteile in die Aktivatorlösung ganz oder teilweise eingetaucht werden.

10. Verwendung der Aktivatorlösung nach Anspruch 1 bis 5 zum beschleunigten Aushärte von Cyanacrylat Klebstoffen.

## Claims

1. An activator solution for the accelerated curing of cyanoacrylate adhesives, **characterised in that** the activator is dissolved in a solvent with a boiling point below 120°C and the activator is an organic compound containing the structural element -N=C-S-S-.

2. The activator solution according to claim 1, **characterised in that** the organic compound contains the structural element -N=C-S-S-C=N-.

3. The activator solution according to claim 2, **characterised in that** the activator is selected from dibenzothiazyl disulfide, 6,6'-dithiodinicotinic acid, 2,2'-dipyridyl disulfide, bis-(4-t-butyl-1-isopropyl-2-imidazolyl) disulfide.

4. The activator solution according to at least one of the preceding claims, **characterised in that** volatile hydrocarbons, carboxylic acid esters, ketones, ethers or halogenated hydrocarbons are used as the solvent.

5. The activator solution according to at least one of the preceding claims, **characterised by** an activator concentration from 0.01 g to 10 g, preferably 0.1 g to 5 g of activator substance per 100 ml of solvent.

6. A method for adhesively bonding substrates, **characterised by** the following essential process steps:
a) spraying the activator solution according to claim 1 to 5 onto at least one adherend surface of the substrates or parts to be joined
b) allowing the solvent to evaporate, optionally by heating or using a blower
c) applying the cyanoacrylate adhesive to at least one substrate part
d) bringing together the substrate parts to be joined, optionally fixing them in position mechanically in the short term
e) optionally subsequently spraying the activator solution according to claim 1 to 5 onto exposed adhesive or adhesive used in excess or adhesive emerging from the joint gap.

7. The method according to claim 6, but without step b).

8. The method according to claims 6 or 7, with the difference that in step a) the activator solution is applied to the parts to be joined in advance using a brush or stirring rod or spatula.

9. The method according to claim 8, with the difference that in step a) the parts to be adhesively joined are partially or wholly immersed in the activator solution.

10. Use of the activator solution according to claim 1 to 5 for the accelerated curing of cyanoacrylate adhesives.

## Revendications

1. Solution d'activateur pour le durcissement accéléré d'adhésifs au cyanoacrylate, **caractérisée en ce que** l'activateur est dissous dans un solvant présentant un point d'ébullition inférieur à 120°C et l'activateur est un composé organique qui contient l'élément structurel -N=C-S-S-.

2. Solution d'activateur selon la revendication 1, **caractérisée en ce que** le composé organique contient l'élément structurel -N=C-S-S-C=N-.

3. Solution d'activateur selon la revendication 2, **caractérisée en ce que** l'activateur est choisi parmi le disulfure de dibenzothiazyle, l'acide 6,6'-dithiodinicotinique, le disulfure de 2,2'-dipyridyle, le disulfure de bis-(4-t-butyl-1-isopropyl-2-imidazolyle).

4. Solution d'activateur selon au moins l'une quelconque des revendications précédentes, **caractérisée en ce qu'**on utilise, comme solvants, des hydrocarbures volatils, des esters d'acide carboxylique, des cétones, des éthers ou des hydrocarbures halogénés.

5. Solution d'activateur selon au moins l'une quelconque des revendications précédentes, **caractérisée par** une concentration en activateur de 0,01 g à 10 g, de préférence de 0,1 g à 5 g de substance d'activateur par 100 ml de solvant.

6. Procédé pour le collage de substrats, **caractérisé par** les étapes de procédé essentielles suivantes
a) pulvérisation de la solution d'activateur selon la revendication 1 à 5 sur au moins une surface à coller des substrats ou des pièces à assembler
b) évaporation du solvant, le cas échéant en chauffant ou à l'aide d'un ventilateur
c) application de l'adhésif au cyanoacrylate sur au moins une partie du substrat
d) assemblage des pièces de substrat à assembler, le cas échéant avec une fixation mécanique pendant un court laps de temps
e) le cas échéant pulvérisation ultérieure de la solution d'activateur selon la revendication 1 à 5 sur l'adhésif qui dépasse ou utilisé en excès ou sortant de la fente d'assemblage.

7. Procédé selon la revendication 6, cependant sans l'étape b).

8. Procédé selon les revendications 6 ou 7, avec comme différence que dans l'étape a), la solution d'activateur est appliquée au préalable à l'aide d'un pinceau ou d'une tige d'agitation ou d'une spatule sur les pièces à assembler.

9. Procédé selon la revendication 8, avec comme différence que dans l'étape a), les pièces à assembler par collage sont totalement ou partiellement plongées dans la solution d'activateur.

10. Utilisation de la solution d'activateur selon la revendication 1 à 5 pour le durcissement accéléré d'adhésifs au cyanoacrylate.
